# EUROPEAN PATENT APPLICATION

(11) **EP 0 533 409 A1**
(43) Date of publication of application: **24.03.1993**
(21) Application number: 92308284.6
(22) Date of filing: 11.09.1992
(51) Int. Cl.: A61M 15/00, A61M 16/10

(54) **Method and apparatus for administering respirable pharmaceutical particles**

(30) Priority: 19.09.1991 GB 9120013
(71) Applicant: THE WELLCOME FOUNDATION LIMITED, London NW1 2BP (GB)
(72) Inventor: Brown, David Leslie, North Carolina 27243, Orange County (US); Davis, Craig William, North Carolina 27858, Pitt County (US); Pattishall, Edward Nolan, North Carolina 27516, Orange County (US); Staton, Jeffrey Scott, North Carolina 27884, Pitt County (US); Zaccardelli, David Scott, North Carolina 27513, Wake County (US)
(74) Representative: Stott, Michael John

(57) **Abstract**

An apparatus for administering respirable pharmaceutical particles to at least one lung of a patient is disclosed. The apparatus comprises a ventilator (10) for cyclically forcing oxygen-rich air into at least one lung of the patient and collecting oxygen-depleted air exhaled by the patient. An inspiratory limb (30) is connected to the ventilator for carrying the oxygen-rich air from the ventilator to the patient, and an expiratory limb (40) is connected to the ventilator for carrying the oxygen-depleted air exhaled by the patient back to the ventilator. A Nebulizer (20) is connected to the inspiratory limb for introducing respirable pharmaceutical particles into the oxygen-rich air. A prefilter (42) is connected to the expiratory limb between the patient and the ventilator for removing respirable pharmaceutical particles from the oxygen-depleted air exhaled by the patient before it is returned to the ventilator.

## Description

The present invention concerns a method and apparatus for filtering aerosol particles, such as surfactant particles, from the expiratory limb of a ventilating apparatus following the administration of the aerosol particles to the lungs of a patient.

Respiratory distress syndrome (RDS), also termed hyaline membrane disease, is the leading cause of death and disability among premature infants. Of the 230,000 to 250,000 infants born prematurely each year in the United States, 40,000 to 50,000 develop RDS; and of those who develop this disease, 5,000 to 8,000 die. See generally R. Perelman and P. Farrell, Pediatrics 70, 570 (1982); D. Vidyasagar, in Hyaline Membrane Disease: Pathogenesis and Pathophysiology, 98 (L. Stern Ed. 1984). In addition, RDS can occur in children, adolescents, and adults as a result of trauma or other injury to the lungs. 150,000 cases of adult respiratory distress syndrome (ARDS) are reported annually, with 60-80% mortality. See American Lung Program, Respiratory Diseases, Task Force Report on Problems, Research Approaches, and Needs, National Heart and Lung Institute, DHEW Publn. (NIH) 73-432: 165-80 (1972).

RDS is caused by a primary deficiency in lung surfactant, a material ordinarily secreted onto the surface of lung alveoli. ARDS consists of a secondary deficiency in lung surfactant due to surfactant inhibition and/or decreased secretion. In the absence of surfactant, the alveoli tend to collapse during exhalation. Collapse can be avoided by mechanically ventilating the lungs. A problem with mechanical ventilation, however, is that it can cause damage to the lungs because of high oxygen concentrations and positive pressures.

A number of groups have sought to develop surfactant formulations which can be used to treat or prevent RDS and ARDS. Both human and bovine natural surfactants have been administered into the airways of human subjects. See, e.g., J. Horbar et al., N. Eng. J. Med. 320, 959 (1989); R. Soll et al., Pediatric Res. 23, 425A (1988). Problems with such natural surfactants are, however, potential contamination with microorganisms and potential sensitization of the patient to proteins therein. Accordingly, completely synthetic surfactants have been developed. See, e.g., U.S. Patent No. 4,826,821 to Clements; U.S. Patent No. 4,312,860 to Clements.

While the development of surfactant formulations have provided an alternative to mechanical ventilation alone, clinicians are now faced with the difficult problem of how to quickly and efficaciously administer these formulations to the lungs of patients. U.S. Patent No. 4,832,012 to Raabe and Lee discloses a nebulizing apparatus which may be used to deliver respirable pharmaceutical particles to the lungs of patients in in conjunction with a ventilating apparatus. Nothing in this disclousre suggests that respirable pharmaceutical particles might be found in the expiratory limb of the ventilating apparatus, or how to obviate this problem if encountered.

A first aspect of the present invention is an apparatus for administering respirable pharmaceutical particles to at least one lung of a patient. The apparatus comprises a ventilator for cyclically forcing oxygen-rich air into at least one lung of the patient and collecting oxygen-depleted air exhaled by the patient. An inspiratory limb is connected to the ventilator for carrying the oxygen-rich air from the ventilator to the patient, and an expiratory limb is connected to the ventilator for carrying the oxygen-depleted air exhaled by the patient back to the ventilator. A Nebulizer is connected to the inspiratory limb for introducing respirable pharmaceutical particles into the oxygen-rich air. A prefilter is connected to the expiratory limb between the patient and the ventilator for removing respirable pharmaceutical particles from the oxygen-depleted air exhaled by the patient. In a preferred embodiment, a bacterial filter is connected to the expiratory limb between the prefilter and the ventilator.

A second aspect of the present invention is a method of administering respirable pharmaceutical particles to at least one lung of a patient. The method comprises providing a ventilator for cyclically forcing oxygen-rich air into at least one lung of the patient and collecting oxygen-depleted air exhaled by the patient. Respirable pharmaceutical particles are introduced into the oxygen-rich air, and respirable pharmaceutical particles are removed from the oxygen-depleted air exhaled by the patient (preferably by passing the oxygen-depleted air through a deep-bed filter) before the oxygen-depleted air is returned to the ventilator. In a preferred embodiment, bacteria are filtered from the oxygen-depleted air after the step of removing respirable pharmaceutical particles, but before the oxygen-depleted air is returned to the ventilator.

A further aspect of the present invention is an apparatus for administering respirable pharmaceutical particles to at least one lung of a patient, comprising: a ventilator for cyclically forcing oxygen-rich air into at least one lung of said patient, and collecting oxygen-depleted air exhaled by said patient; an inspiratory limb having a proximal end and a distal end, said inspiratory limb proximal end connected to said ventilating means for carrying said oxygen-rich air from said ventilator to said patient; an expiratory limb having a proximal end and a distal end, said expiratory limb proximal end connected to said ventilating means for carrying said oxygen-depleted air exhaled by said patient to said ventilator; junction means connected to said inspiratory limb distal end and to said expiratory limb distal end for administering said oxygen-rich air to said patient and receiving said oxygen-depleted air exhaled by said patient; a nebulizer connected to said inspiratory limb between said ventilator and said junction means for loading oxygen-rich air containing respirable pharmaceutical particles into said inspiratory limb on receipt of an activating signal; and trigger means interconnecting said ventilator and said nebulizer for activating said nebulizer while said ventilator is collecting oxygen-depleted air exhaled by said patient.

A further aspect of the present invention is a method of administering respirable pharmaceutical particles to at least one lung of a patient through the inspiratory limb of a ventilator, the inspiratory limb including a distal portion adjacent the patient, with the ventilator cyclically forcing oxygen-rich air into at least one lung of the patient and permitting oxygen-depleted air to be exhaled by the patient, said method comprising: loading the inspiratory limb distal portion with a charge volume of oxygen-rich air containing respirable pharmaceutical particles while oxygen-depleted air is being exhaled by said patient; and then forcing oxygen-rich air into said inspiratory limb distal portion so that said charge volume is forced into at least one lung of said patient, whereby said respirable pharmaceutical particles are administered to the patient.

The foregoing and other aspects of the present invention are explained in detail in the drawings herein and specification set forth below.

The terms "ventilate", "ventilating" and "ventilation" herein refer to the process of cyclically forcing oxygen-rich air into, and permitting oxygen-depleted air to escape from, at least one lung of a patient to assist the breathing of the patient or to breathe for the patient when the patient would not otherwise breathe on its own. Ventilation may be of either or both lungs of a patient, depending on whether any one lung is blocked, collapsed, or otherwise inoperable. Numerous types of ventilating apparatus are known. When respirable particles are to be administered to a patient by ventilation, the respirable particles are typically generated by means of a nebulizer, with the nebulizer output combined with or delivered to the ventilator output for ultimate delivery to the patient. The nebulizer can load the ventilator during the inhalation phase or exhalation phase of the ventilator cycle.

Any nebulizing apparatus capable of forming respirable particles (e.g, particles from .5 to 10 microns, or more particularly .5 to 5 microns, volume median diameter (or "VMD") in size) from a liquid pharmaceutical formulation as described below, may be employed in practicing the present invention, including jet nebulizers, ultrasonic nebulizers, and metered dose inhalers. Those skilled in the art will appreciate that the aerosol produced by the nebulizing apparatus may contain larger particles which are not respirable, so long as a sufficient quantity of respirable particles are included in the aerosol to accomplish the intended purpose. An example jet nebulizer is described in U.S. Patent No. 4,832,012. Example ultrasonic nebulizers include the Porta-Sonic^{TM} and the Pulmo-Sonic^{TM} nebulizers produced by The DeVilbiss Co., Somerset, Pennsylvania, USA. In general, the nebulizer should be capable of introducing from 5 to 50 mL of respirable pharmaceutical particles as an aerosol into each liter of oxygen-rich air administered to the patient.

One particular apparatus for practicing the present invention comprises a ventilator for cyclically forcing oxygen-rich air into at least one lung of the patient, and collecting oxygen-depleted air exhaled by the patient (the term "oxygen-depleted" refering to the reduced oxygen content of exhaled air). An inspiratory limb having a proximal end and a distal end is provided, with the inspiratory limb proximal end connected to the ventilator, for carrying the oxygen-rich air from the ventilator to the patient. An expiratory limb having a proximal end and a distal end is also provided, with the expiratory limb proximal end connected to the ventilator for returning the oxygen-depleted air exhaled by the patient to the ventilator. A patient "Y" junction serves as a patient junction means connected to the inspiratory limb distal end and to the expiratory limb distal end for administering the oxygen-rich air to the patient and receiving the oxygen-depleted air exhaled by the patient. A nebulizer is connected to the inspiratory limb between the ventilating means and the junction means for loading oxygen-rich air containing respirable pharmaceutical particles into the inspiratory limb on receipt of an activating signal. A trigger line electrically interconnecting the ventilator and the nebulizer activates the nebulizer during the exhalation phase of the ventilating cycle, while the ventilator is receiving oxygen-depleted air exhaled by the patient. Aerosol particles loaded into the inspiratory limb in this manner are then blown into the patient during the next inhalation cycle, as explained in greater detail below.

The amount of oxygen-rich air containing respirable pharmaceutical particles loaded into the inspiratory limb (or "charging volume") should be a predetermined quantity less than or equal to the tidal volume of the patient being treated. For example, a group of predetermined volumes may be preset on the apparatus (e.g., volumes of 600, 400, 200, and 100 cubic centimeters), and that volume which is less than or equal to the tidal volume of the patient undergoing treatment chosen as the charging volume. This prevents excess aerosol from blowing past the patient during exhalation and unnecessarily loading any filters. In addition, the portion of the inspiratory limb between the intermediate junction and the patient, which may be referred to as the distal portion of the inspiratory limb, preferably has a volume essentially the same as the charging volume. This maximizes dosage delivered to the patient, while again serving to reduce excess aerosol from blowing past the patient and unnecessarily loading any downstream filter. A set of interchangeable inspiratory limb distal portions of different volume, each with a volume appropriate for a different charging volume, may be included with the apparatus for this purpose.

While the foregoing apparatus loads aerosol particles during the exhalation cycle of the ventilator, an apparatus which loads aerosol particles during the inhalation cycle could also be employed.

Prefilters employed in the present invention should have a resistance of not more than 4, and preferably not more than 2, centimeters of water at an air flow of 100 liters per minute, should be capable of collecting respirable particles (i.e., particles in the range of 1 to 5 microns in diameter (VMD)), and should be operable with an air flow having the cyclical velocity profile of a human exhalation (e.g., 50-150 liters per minute, approximately 20 breaths per minute). Suitable prefilters are deep bed filters which provide a tortuous path for air flow through the filter media, and with air flow being substantially uniform through all portions of the filter bed, as opposed to pleated or accordion-folded filters. Particularly preferred are deep-bed fiber filters and deep-bed foam filters (including combinations thereof in which both fiber and foam layers contacting one another are included).

Deep-bed fiber filters typically have filter beds of from about .5 to 5 centimeters thick, and may be made from any suitable fiber material, including both hydrophobic and hydrophilic materials. Suitable fiber materials include, for example, metals such as stainless steel, titanium, etc.; polymeric materials such as polyesters, polyvinylchloride, polyethylene terephthalate, fluorocarbons such as Teflon, nylons, polyalkylenes such as polyethylene and polypropylene, etc.; glass such as borosilicate glass;etc. (see, e.g., U.S. Patent No. 4,086,070). Fibers may be arranged in any suitable manner which provides for uniform air flow through the filter bed, such as a packed bed of loose fibers, a wound bed of textile roving, or a wrapped bed of non-woven textile fabric. Fibers may be treated, such as chemically or by heat, to bond fibers to one another, to alter the shape or memory of the fibers, or to render the fibers hydrophobic or hydrophilic. Suitable average fiber diameters are from 1 to 50 microns; and suitable fiber bed voidages (bed volume occupied by fiber/total bed volume x 100) range from about 85 to 98 percent.

Deep-bed foam filters may be made of porous, open-celled foam material. A number of foamed plastic materials can be employed for this purpose, one of which is a polyurethane foam having approximately 100 pores per linear inch and an average cell size diameter of about 150 microns (see, e.g., U.S. Patent No. 3,802,160 at Col. 4 lines 10-25).

Any bacterial filter may be employed in the expiratory limb of the apparatus of the present invention, with the bacterial filter positioned between the prefilter and the ventilator. Currently preferred is the STAR^{TM} main flow bacterial filter, which is available from Infrasonics, Inc. (San Diego, California). The bacterial filter should have a resistance of not more than 4 centimeters of water at an air flow of 100 liters per minute. In a preferred embodiment of the invention, the total resistance of the expiratory limb, including both prefilter and bacterial filter (when present), is not more than 4 centimeters of water at an air flow of 100 liters per minute.

While the present invention is primarily contemplated for use in administering surfactant formulations, any pharmaceutical formulation may be administered as respirable aerosol particles by the method and apparatus of the present invention. Exemplary pharmaceutical formulations which may be administered to the lungs include, but are not limited to, formulations containing β-2 agonists (e.g., epinephrine), pentamidine, ribavirin, atropine, and acetylcholine. In pharmaceutical formulations the active ingredient is normally provided in a pharmaceutically acceptable carrier, examples of which include, but are not limited to, sterile pyrogen-free water and physiological saline solution.

Surfactant formulations used in practicing the present invention may be of any type useful for the treatment of RDS or ARDS, whether of natural (i.e., human, bovine), see, e.g., J. Horbar et al., N. Eng. J. Med. 320, 959 (1989); R. Soll et al., Pediatric Res. 23, 425A (1988), recombinant, or synthetic origin, or combinations thereof. See, e.g., Y. Tanaka et al., J. Lipid Res. 27, No. 2, 475 (1986), T. Fujiwara et al., Lancet 1, 55 (January 12, 1980) (cow-lung extract fortified with dipalmitoylphosphatidylcholine); U.S. Patent No. 4,912,038 to Schilling et al. (recombinant DNA sequences encoding alveolar surfactant proteins). Particularly preferred for practicing the present invention is synthetic surfactant of the type described in U.S. Patent No. 4,826,821 to Clements (Applicants specifically intend that the disclosure of all patent references cited herein be incorporated herein by reference). Also useful for practicing the present invention is synthetic surfactant of the type described in U.S. Patent No. 4,312,860 to Clements. Another surfactant formulation is commercially available from Ross Laboratories as SURVANTA^{R}, which is a natural bovine lung extract containing phospholipids, neutral lipids, fatty acids, and surfactant-associated proteins to which dipalmitoylphosphatidylcholine, palmitic acid, and tripalmitin are added to standardize the composition and to mimic surface tension lowering properties of natural lung surfactant. The surfactant formulation may be provided as a sterile lyophylized powder, which is reconstituted prior to use, or as a ready-to-use liquid suspension.

In general, all surfactant formulations contain dipalmitoylphosphatidylcholine (DPPC; also called "Colfosceril Palmitate") as a phospholipid in an aqueous carrier, either alone or in combination with other phospholipids such as 1-palmitoyl-2-oleoyl-phosphatidylglycerol, dimyristoyl phosphatidylcholine, distearoyl phosphatidylcholine, dimyristoyl phosphatidylethanolamine, dilauroyl phosphatidylethanolamine, dimethyl dipalmitoyl phosphatidylcholine, methyl dipalmitoyl phosphatidylcholine, dipalmitoyl phosphatidylglycerol, phosphatidylcholine, dipalmitoyl phosphatidyl ethanolamine, dilauroyl phosphatidylcholine, dioleoyl phosphatidylcholine, and dibehenoyl phosphatidylcholine. Typically, DPPC is included in an amount from 10 to 90 mg/mL in a surfactant formulation to be nebulized and administered to the lungs.

Surfactant formulations typically have a surface tension less than 15 dynes per centimeter. Lower surface tensions (e.g., less than about 10 dynes per centimeter) tend to be preferred, with the surfactant formulations described herein being capable of providing surface tensions down to about 1 to 2 dynes per centimeter or less.

Surfactant formulations preferably include a spreading agent such as a fatty alcohol or a lung surfactant protein in an amount effective to spread the surfactant formulation on the surface of lung alveoli. In a particularly preferred embodiment of the present invention the spreading agent is cetyl alcohol (also called hexadecanol).

Suitable surfactant formulations include those containing a lung surfactant protein as the spreading agent, as noted above. An example of such a formulation is prepared by combining a surfactant apoprotein such as described in U.S. Patent No. 4,912,038 with a mixture of lipids, where the mixture of lipids includes DPPC, 1-palmitoyl-2-oleoyl-phosphatidylglycerol (POPG), and palmitic acid (PA), with the ratio of DPPC to POPG being from 50:50 to 90:10; with the ratio of (DPPC + POPG) to surfactant protein being from 50:1 to 5:1; and with the ratio of PA to (DPPC + POPG) being from 0 to 0.2. Other unsaturated acidic lipids than POPG may be substituted for POPG. In a particularly preferred embodiment of this type of formulation, the ratio of DPPC to POPG to PA is 7:3:1, with a final phospholipid concentration of 3 times the amount of lipid, with calcium incorporated at approximately 0.01mg/mL to 10mg/mL Ca²⁺. See, e.g., PCT Application Publication No. WO 91/00871 of Genentech Inc. and California Biotechnology Inc. (Published 24 Jan 1991).

The dispersion is preferably heated to a temperature between about 25°C and 75°C, and the phospholipid is preferably dipalmitoylphosphatidylcholine (DPPC) included in an amount from about 30 to 90 milligrams per milliliter of aqueous carrier. Various combinations of heating and phospholipid concentration may be employed in practicing the present invention, with the following embodiments employing DPPC being illustrative:
(1) A method wherein the dispersion is heated to a temperature of about 45°C to 75°C and the DPPC is included in the aqueous carrier in an amount from about 5 to 24 milligrams per milliliter (mg/mL). Most preferably, the dispersion is heated to a temperature of about 50°C and the DPPC is included in an amount of about 13.5mg/mL.
(2) A method wherein the dispersion is heated to a temperature of about 45°C to 75°C and the DPPC is included in the aqueous carrier in an amount from about 30 to 50mg/mL. Most preferably, the dispersion is heated to a temperature of about 50°C and the DPPC is included in an amount of about 40.5mg/mL.
(3) A method wherein the dispersion is heated to a temperature of about 45°C to 75°C, and the DPPC is included in the aqueous carrier in an amount from about 70 to 90mg/mL. Most preferably, the dispersion is heated to a temperature of about 50°C and the DPPC is included in an amount of about 81mg/mL. In general, the dispersion is heated to a temperature of at least about the transition temperature of the phospholipid. Note that nebulization of the surfactant formulation causes the temperature of that formulation to drop, so heating the formulation will be required to maintain the temperature of the surfactant formulation even at room temperature during continuous nebulization.

The present invention is explained in greater detail in the examples set forth below.

### EXAMPLE 1 Preparation of Surfactant Formulation

25 cc of sterile, pyrogen-free water is taken up in a sterile disposable syringe and injected into a sterile, evacuated, 50cc vial containing a lyophilized powder of 2.025g dipalmitoylphosphatidylcholine (DPPC), 225mg hexadecanol, 150mg tyloxapol, and 876.6mg NaCl. This mixture is known. See U.S. Patent No. 4,826,821 to Clements. The water and powder are vigorously mixed by inverting the vial and repeatedly withdrawing the mixture into the syringe and releasing the plunger of the syringe. The result is approximately 25cc of surfactant formulation, which is a suspension rather than a solution. The formulation is placed in a nebulizer well and diluted as needed with 125mL of sterile pyrogen free water. The resulting surfactant formulation is 0.1N with respect to NaCl, has an osmolality of 190mOsm/l, and contains 13.5mg/cc of DPPC, 1.5mg/cc of hexadecanol, and 1mg/cc of tyloxapol. This formulation is referred to herein as a 1X surfactant formulation. A 3X and 6X surfactant formulation is prepared in essentially the same manner, except that the DPPC, hexadecanol, and tyloxapol are increased three and six times by weight, respectively. NaCl is maintained at approximately a 0.1 N concentration in the 3X and 6X formulations.

### EXAMPLE 2 Combination Ventilating and Nebulizing Apparatus

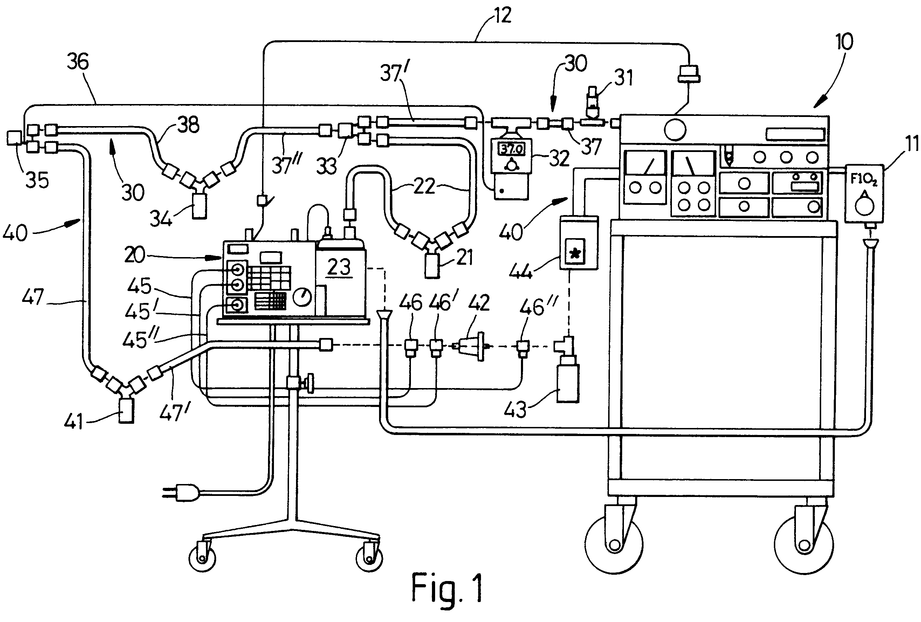

A preferred embodiment of the present invention is illustrated in Figure 1. This apparatus is assembled from a SERVO 900C^{TM} ventilator 10 (Siemens-Elema AB, Sweden) and a VISAN 9^{TM} jet nebulizer unit (20) (Vortran Medical Technology,Inc., Sacramento, California). Compressed air (345 kPa) is supplied to an oxygen/air blender (11) and used to supply gas to both the ventilator (10) and the nebulizer (20), with the nebulizer gas supply passing through a separate nebulizer flowmeter. The nebulizer is electrically connected to the ventilator by line (12), which provides an electrical signal at the onset of the exhalation phase of the breathing cycle so that nebulized surfactant is delivered during the exhalation phase of the ventilator respiratory function, as explained in greater detail below.

The inspiratory limb (30) of the apparatus includes a pressure release valve (31), a humidifier (32), an intermediate "Y" junction (33), a water trap (34), and a patient "Y" junction (35). The humidifier is connected to a remote temperature monitor at the patient "Y" junction by means of an electrical connection (36). All connections are made with flexible tubing (37), (38).

The nebulizer (20) is connected to the intermediate "Y" junction (33) through a water trap (21). All connections are made with flexible tubing (22).

The expiratory limb (40) of the apparatus includes a water trap (41), a deep-bed fiber prefilter (42), a water trap (43), and a STAR^{TM} bacterial filter (44). Air exhaled by the patient is returned to the ventilator 10 for monitoring and measurement through the bacterial filter. Pneumatic lines (45), (45′), (45)˝ connect pressure sensors in the nebulizer to various locations in the expiratory limb through "T" junctions (46), (46)′, (46)˝. Again, all connections are made with flexible tubing (47). The ventilator (10) functions in the usual manner, cyclically forcing oxygen-rich air into at least one lung of the patient, and collecting oxygen-depleted air exhaled by the patient. The number of breaths per minute and the tidal volume of each breath is controlled by the ventilator.

The nebulizer (20) receives the same air supply as the ventilator. The nebulizer includes a container (23) for the surfactant formulation which is heated so the formulation is more rapidly aerosolized and administered to the patient. The nebulizer produces aerosol from the surfactant formulation in an amount of from about 10 to 50mL, or more preferably about 35mL, per liter of air. As noted above, a triggering circuit in the nebulizer is connected to a gate valve in the ventilator by electrical line (12) so that aerosol-laden oxygen-rich air is blown into the inspiratory limb distal portion (38) of the apparatus only during the exhalation cycle of the ventilator. During the exhalation cycle, the gate valve in the inspiratory side of the ventilator is closed and the gate valve in the expiratory side of the ventilator is open so that air exhaled by the patient into the patient "Y" junction (35) travels through the expiratory limb of the circuit. When aerosol-laden oxygen-rich air is blown into the inspiratory- limb distal portion (38) during the exhalation phase, it travels into intermediate "Y" junction (33), down the inspiratory limb to the patient "Y" junction (35), and back up the expiratory limb (40). Aerosol-laden air from the nebulizer does not travel back towards the inspiratory side of the ventilator because the gate valve on this side of the nebulizer is closed, and does not travel into the patient because the patient is exhaling. A predetermined quantity of aerosol-laden air is blown into the inspiratory limb (30) sufficient to fill this limb from the intermediate "Y" junction (33) to the patient "Y" junction, with the volume of this portion of the inspiratory limb (30) being not less than the tidal volume set by the ventilator (10) for the inhalation phase. This maximizes administration of aerosol to the patient. The aerosol-laden oxygen-rich air in this portion of the inspiratory limb (30) is then blown into the patient by the ventilator during the next inhalation phase of the ventilating cycle. The gate valve in the nebulizer (20) is closed during the inhalation phase of the ventilation cycle to prevent air from the ventilator from blowing into the nebulizer.

### EXAMPLE 3 Initial Prefilter Constructions and Results

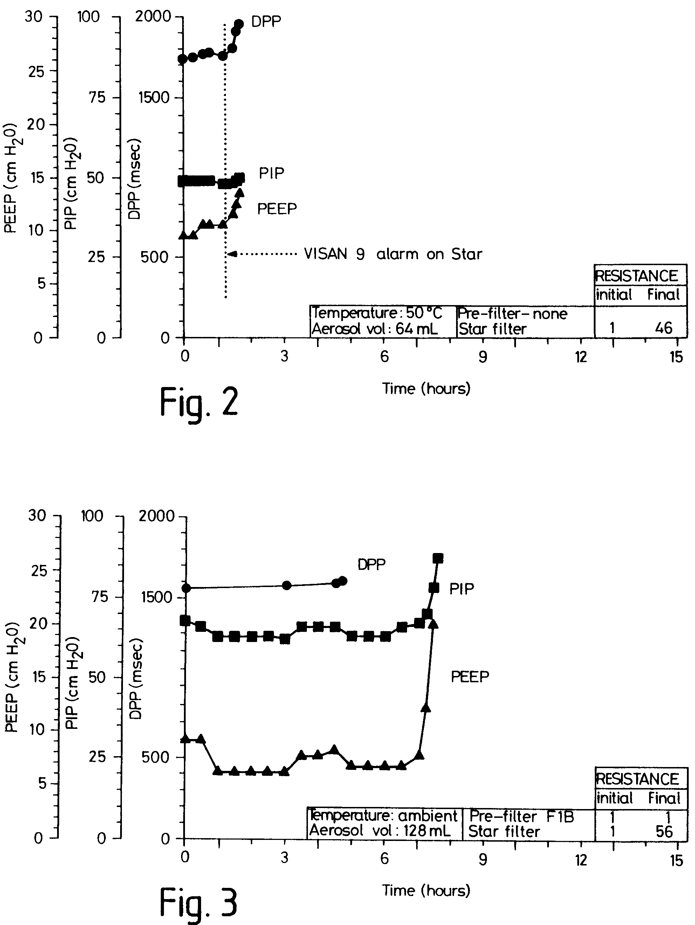

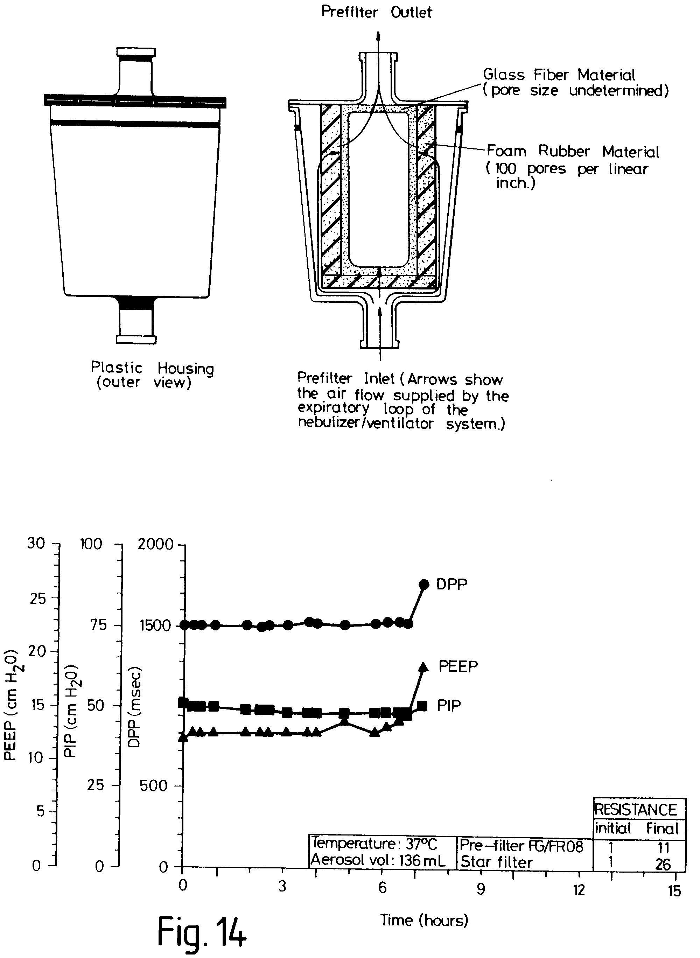

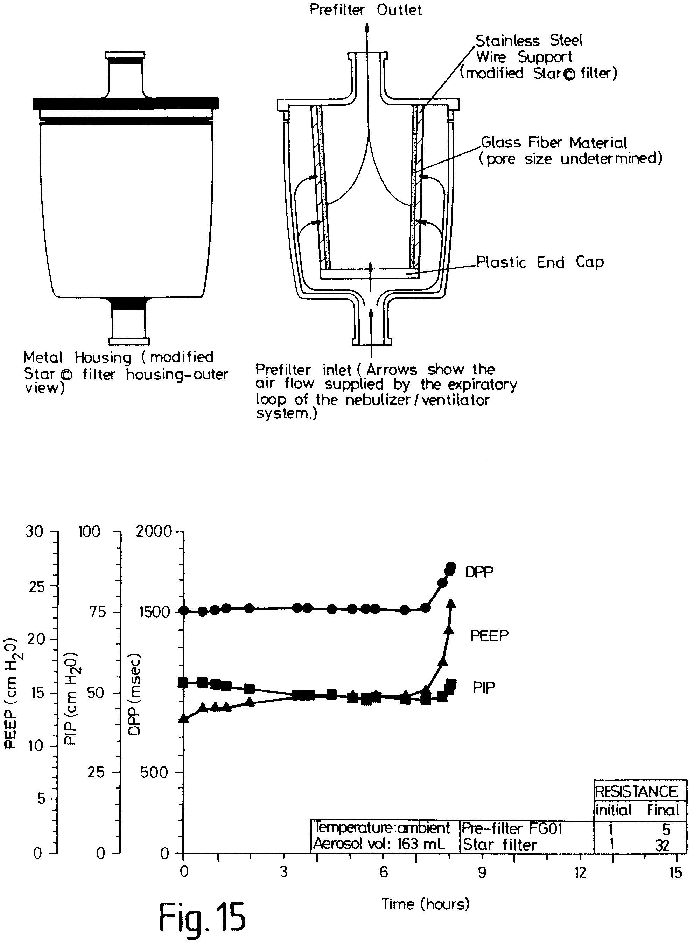

Various prefilter constructions, and the results obtained therewith, are illustrated in Figures 2 through 15. In the graphs presenting data in these Figures, "DPP" means duration of positive pressure, "PIP" means peak inspiratory pressure, "PEEP" means positive end expiratory pressure, and "resistance" is the pressure differential in centimeters of H₂O across the filter at 100 liters per minute of air flow. The surfactant formulation used in these tests was essentially as described in Example 1 above, at a 6X concentration.

The prefilters were placed in the expiratory limb of an apparatus essentially the same as described in Example 2 above, except that aerosol was loaded during the inspiratory phase of the ventilating cycle (fifty percent of inspiratory volume originating from the ventilator and 50 percent of the inspiratory volume originating from the nebulizer) and aerosol was supplied to an artificial lung. In brief, the test apparatus was assembled from a SERVO 900C ventilator and a VISAN^{TM} nebulizer unit. Compressed air (345 kPa) was supplied to an oxygen/air blender and used to supply gas to both the ventilator and the nebulizer, with the nebulizer gas supply passing through a separate nebulizer flowmeter. The nebulizer control unit was electrically connected to the electrical signal source of the respirator which controls the inhalation phase of the breathing cycle so that nebulized surfactant was delivered intermittently, during the inhalation phase of the ventilator respiratory function. The ventilator and nebulizer output were each connected to a patient Y tube, which was in turn connected to three artificial foam-lined rubber lungs connected in parallel.

The ventilator and nebulizer unit were set to deliver a combined total inspiratory tidal volume (Vt) of 750mL/breath. Initial tidal volumes were checked by installing a rubber "lung" at the patient Y tube and observing the appropriate ventilator gauges. Ventilator mean airway pressure (cm water) was monitored and recorded initially and at each sampling time to determine the back pressure on the system. Instrument settings for the ventilator and nebulizer unit were as follows: The ventilator was set to a Working pressure of 70cm water; a breathing rate (f) of 20 breaths/min; an inhalation fraction (Pi) of 25%; and an Inspiratory Minute Volume (MV) of 7.5L/min. The nebulizer was set to a working pressure of 345 kPa; a flow rate (from flowmeter) of 30L/min; and a flow rate (from nebulizer) of 500mL/sec (2 nebulizer jets open).

A preliminary control experiment, the data of which is given in Figure 2, shows that the STAR^{TM} bacterial filter is rapidly occluded in the absence of a prefilter. Occlusion of the bacterial filter is indicated by the vertical line marked "VISAN 9 alarm on Star", which means that the resistance of the bacterial filter, as monitored by the nebulizer, has exceeded permissible levels.

Figure 3 shows results obtained with an foam rubber filter. The temperature of the surfactant formulation in the nebulizer bath and the volume of surfactant nebulized is given in the left box of the graph; the initial and final resistance (in centimeters of water pressure at an air flow of 100 liters per minute) of the prefilter and the STAR^{TM} bacterial filter are given in the center and right boxes of the Figure. No change in resistance for the prefilter and the dramatic increase in resistance for the bacterial filter indicates that the prefilter provided no protection for the bacterial filter.

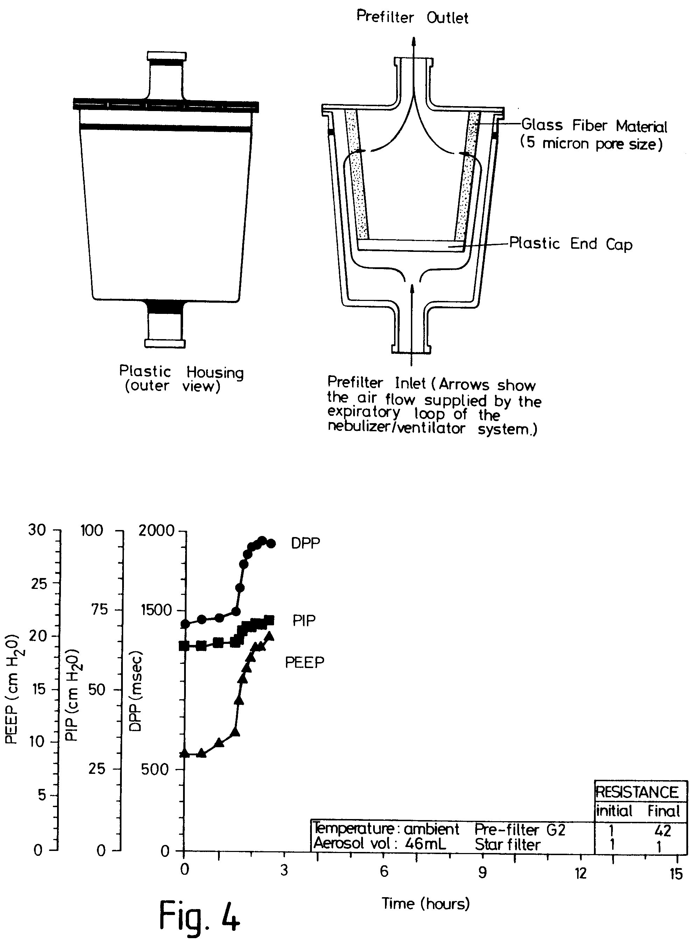

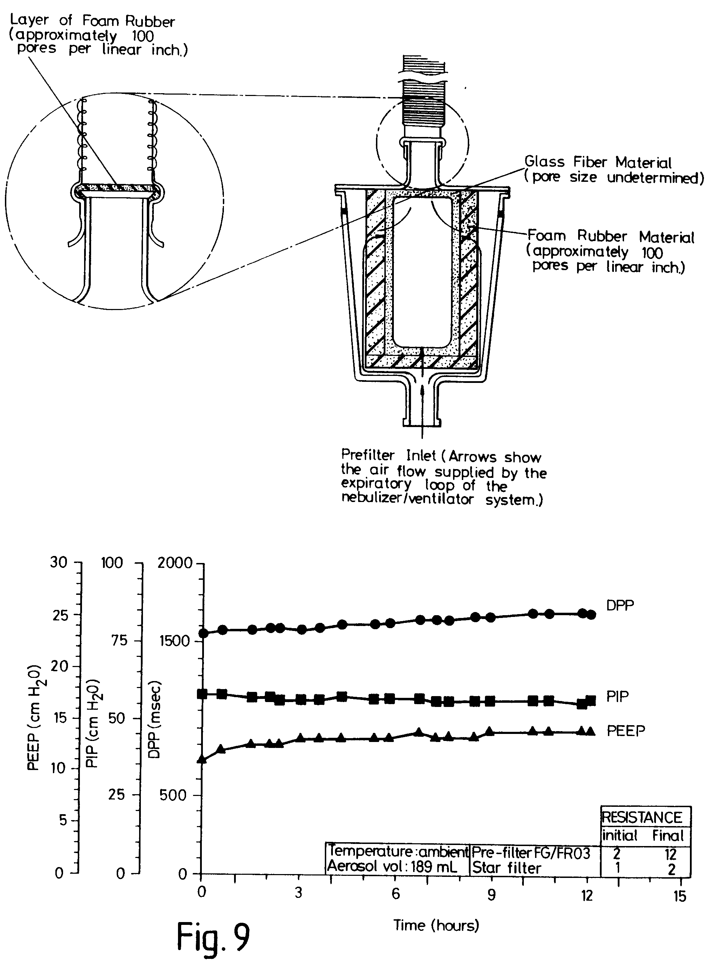

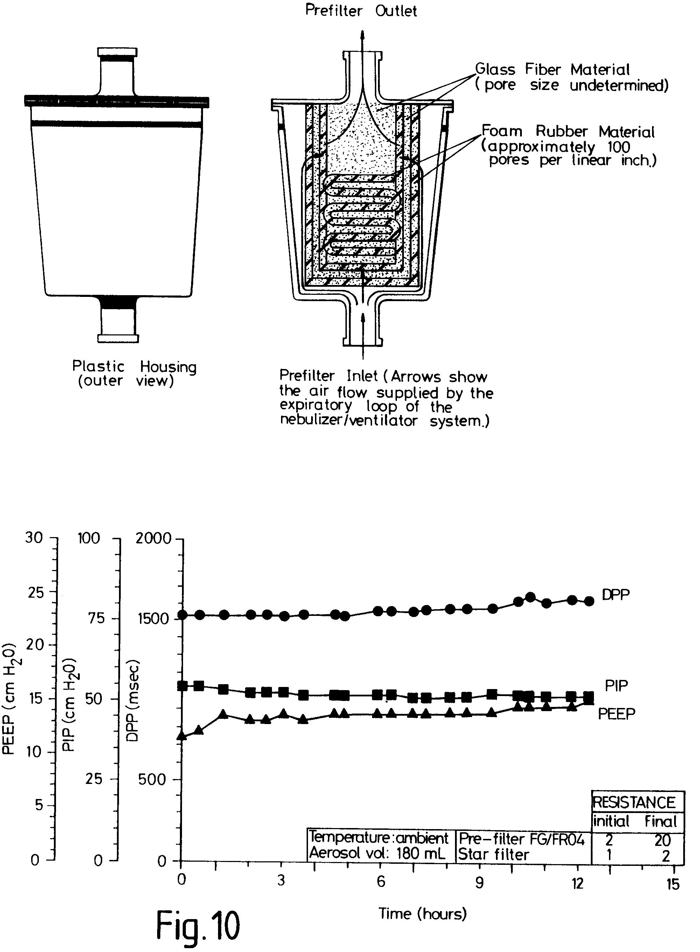

Data for the experiments described in Figures 4 through 15 is given in essentially the same manner as for Figures 2 and 3, except that the prefilter construction employed in each experiment is as illustrated at the top of each figure. The additional porous foam layer illustrated in the experiment of Figure 9 was also employed in the experiments of Figures 10 through 15.

The experiment of Figure 4 employed a glass fiber filter having a nominal pore size of 5 microns as the prefilter. This experiment showed substantially increased protection of the bacterial filter, but a short life-span for the prefilter. Note the small volume of aerosol delivered (46mL).

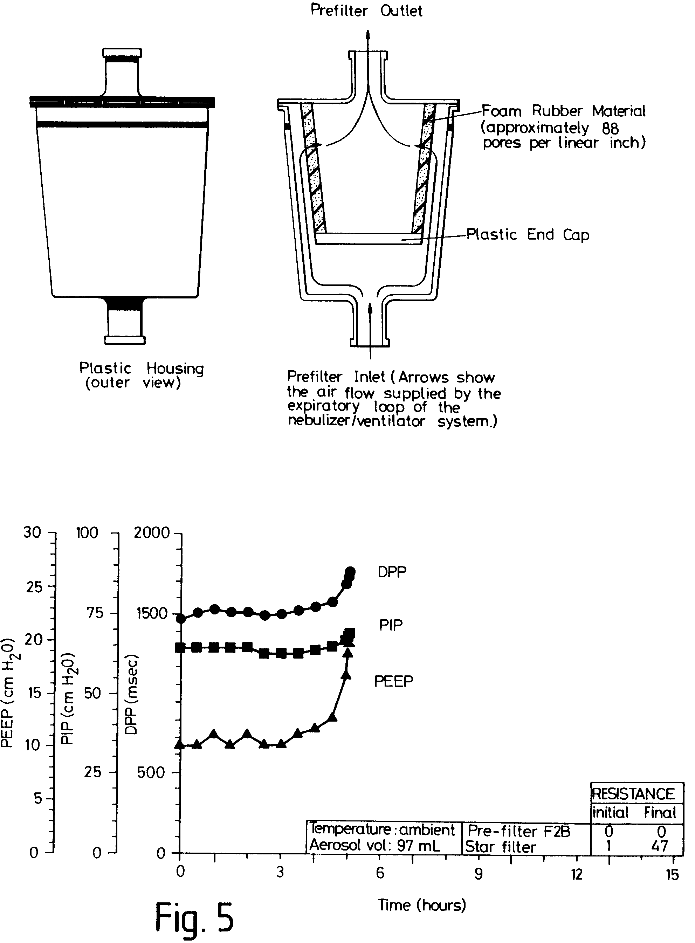

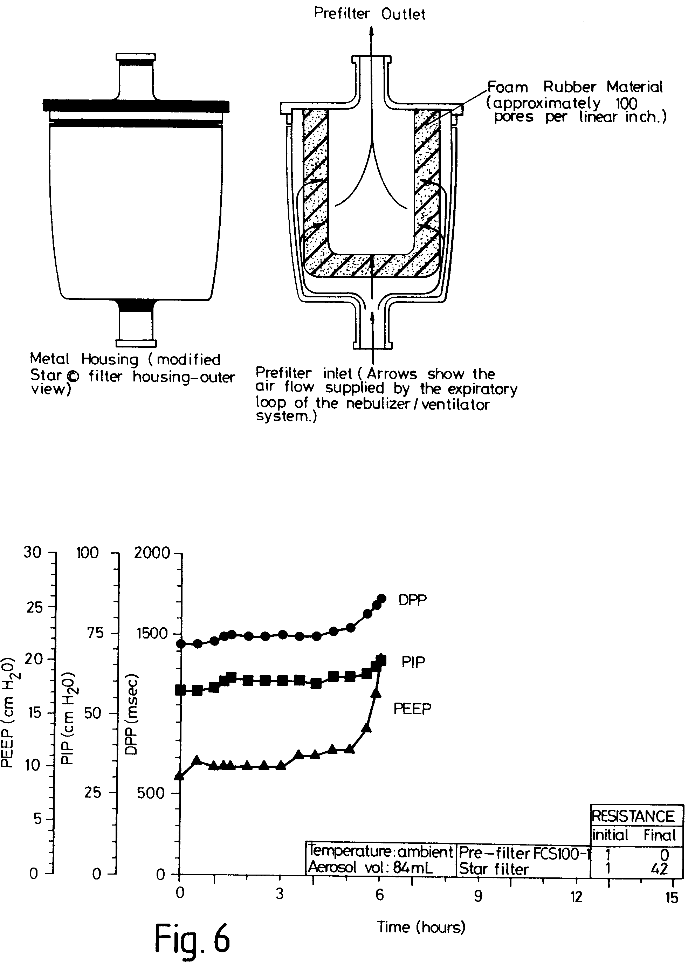

The experiments of Figures 5 and 6 both employed a foam rubber filter Relatively little protection of the bacterial filter was seen.

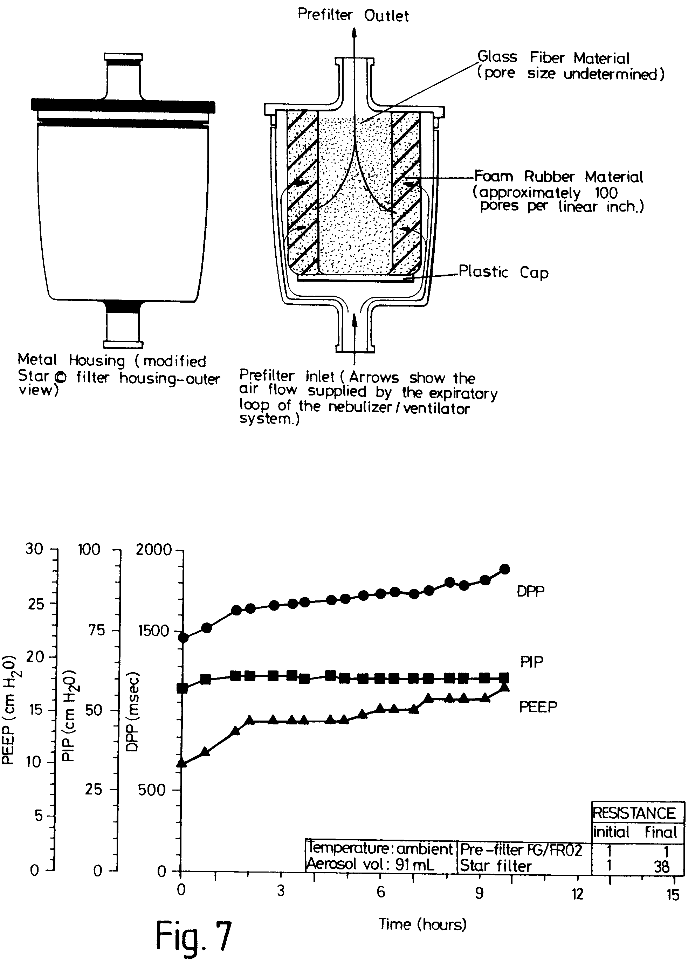

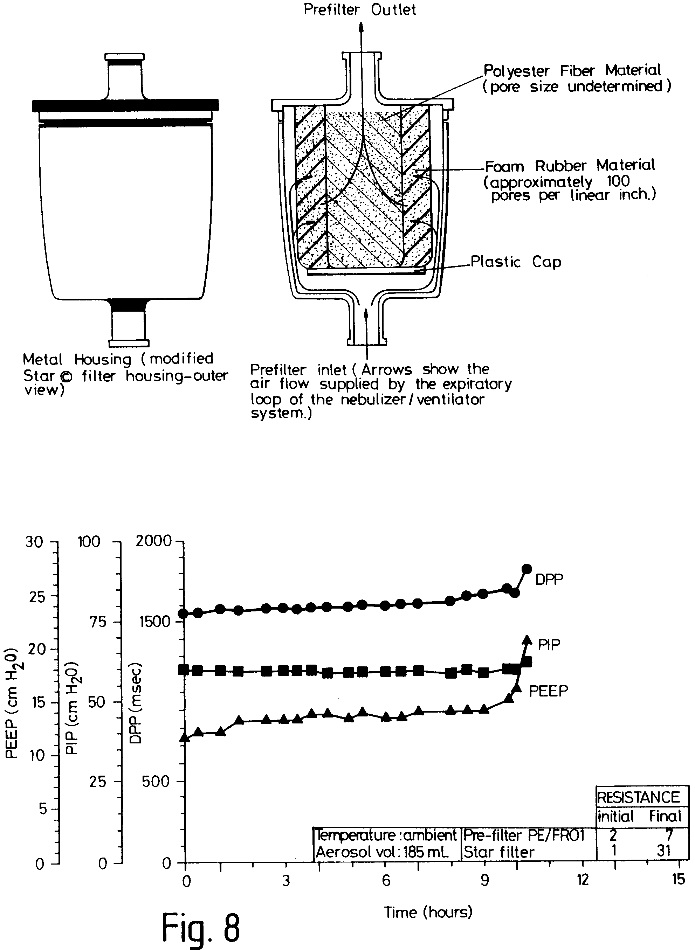

The experiment of Figure 7 employed a filter having a glass fiber filter bed and a foam filter bed in combination. Note that while the bacterial filter eventually became occluded, the lifespan of the bacterial filter was substantially increased. Similar results were seen in the experiment of Figure 8, where a polyester fiber filter bed was combined with the foam filter bed.

The experiments of Figures 9 and 10 show both a long duration of operation and good protection of the bacterial filter.

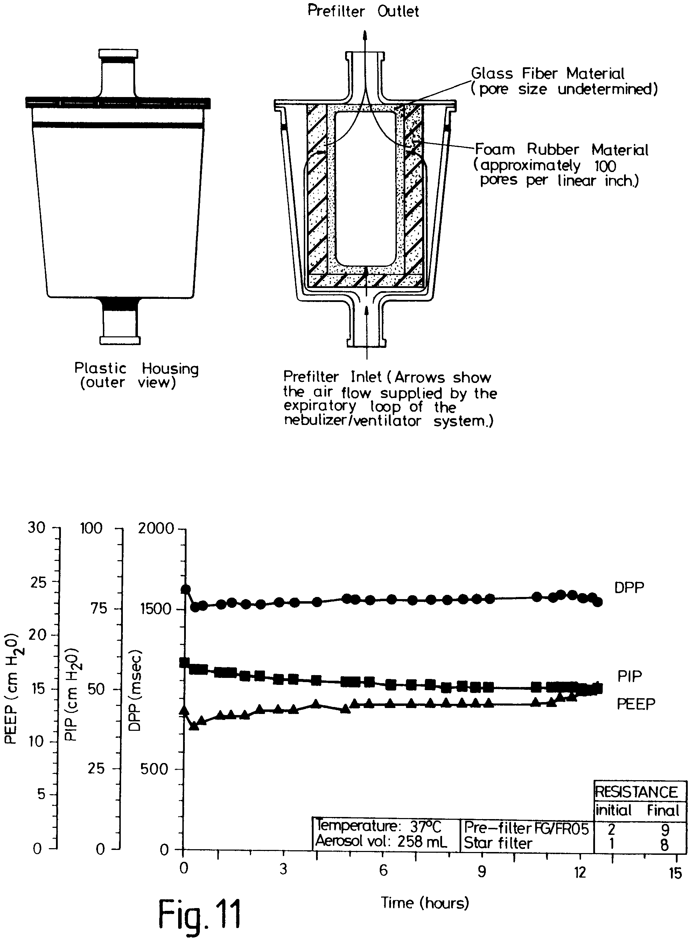

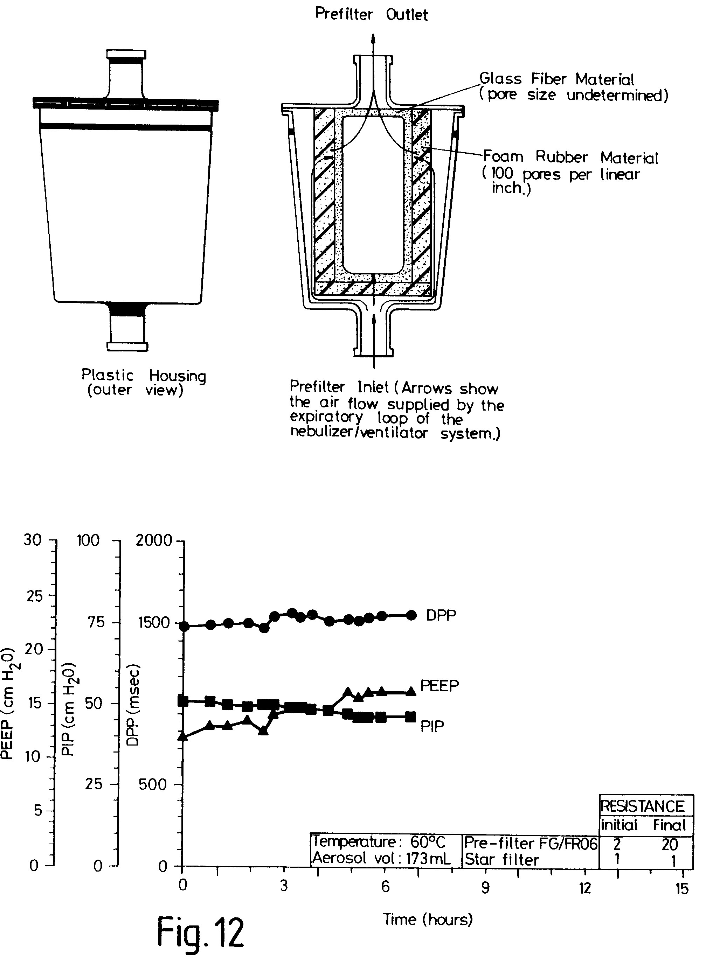

The experiments of Figures 11 and 12 are similar to the experiment of Figure 9, except that the surfactant formulation reservoir in the nebulizer was maintained at a temperature of 37°C and 60°C, respectively. This substantially increased the rate of aerosol delivery. Again, good protection of the bacterial filter was seen.

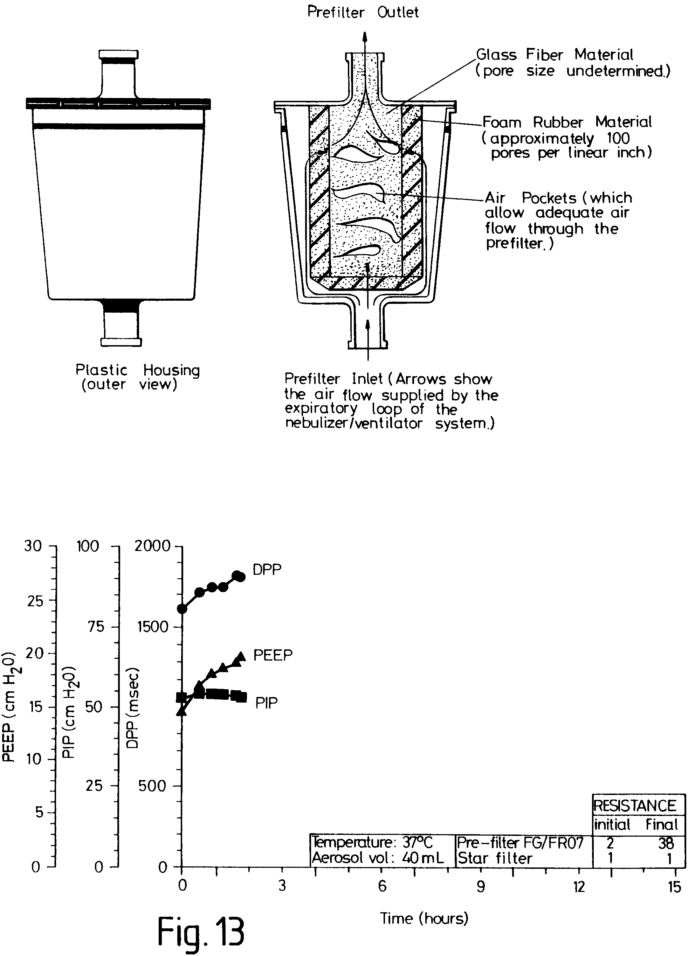

Results obtained with various other filter constructions are illustrated in Figures 13 through 15.

### EXAMPLE 4 Additional Prefilter Testing and Results

This set of experiments is similar to those described in Example 3 above, except experiments were conducted with deep bed fiber filters having pore sizes (nominal at 90% retention efficiency) rated at 1, 3, and 5 microns. The prefilters used were POLYGARD-CR^{TM} polypropylene fiber clarification cartridges, commercially available from Millipore, Inc. (Bedford, Massachusetts, USA).

Figure 16 shows that when a deep bed fiber filter having a nominal pore size of 1 micron is employed as a prefilter, the bacterial filter is completely protected. Note that the temperature of the surfactant reservoir in the nebulizer was maintained at 50°C in this experiment.

Figure 17 shows data from an experiment similar to that illustrated in Figure 16, except a prefilter with a nominal pore size of 3 microns was used. Again, the bacterial filter was protected. Note also that the 3 micron filter had a longer useable lifespan than the 1 micron filter.

Figure 18 shows data similar to Figures 16 and 17, except that two 5 micron nominal pore size filters connected in parallel were used. Note the still greater useable lifespan of the prefilters prior to occlusion of the bacterial filter.

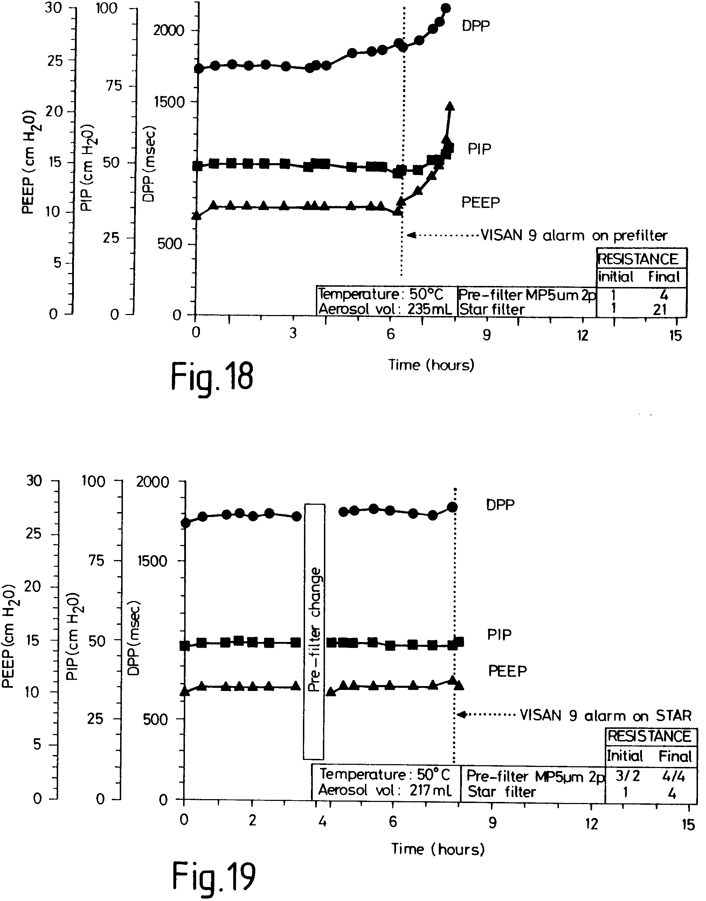

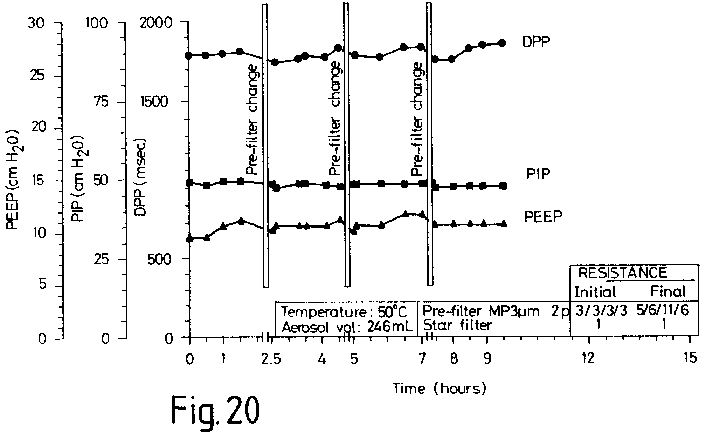

The experiments of Figures 19 and 20 were similar to the experiment of Figure 18, except that prefilters were changed at predetermined intervals, and 3 micron nominal pore size filters were employed in the experiment of Figure 20. Note the long duration of operation, and note that, in the experiment of Figure 20, the bacterial filter appeared completely protected after an extended period of operation.

The foregoing examples are illustrative of the present invention, and are not to be construed as limiting thereof.

The following is a brief description of the Figures as referred to in the foregoing examples.

Figure 1 is an exploded, partially schematic view of a combination ventilating and nebulizing apparatus of the present invention.

Figure 2 illustrates the occlusion of a bacterial filter in the absence of a prefilter in an apparatus similar to that of Fig. 1. "DPP" means duration of positive pressure, "PIP" means peak inspiratory pressure, and "PEEP" means positive end expiratory pressure.

Figure 3 illustrates the protection afforded to a bacterial filter by a porous foam prefilter in an apparatus similar to that shown in Figure 1.

Figures 4 through 15 illustrate, in the same manner as Fig. 3, the protection afforded to a bacterial filter by various alternate constructions of prefilters. The particular construction of prefilter employed is illustrated at the top of each figures.

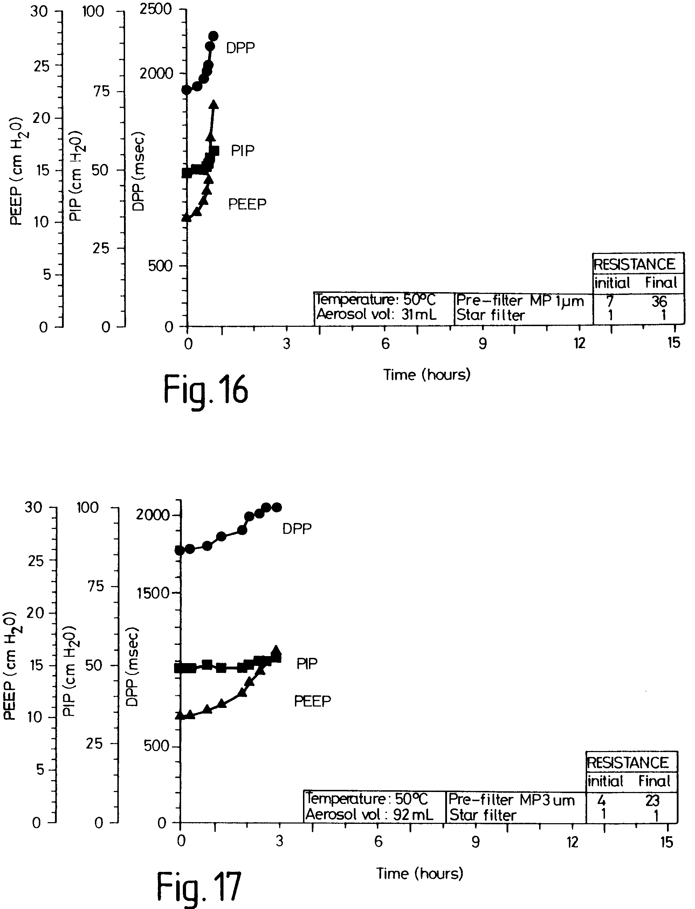

Figures 16 through 20 illustrate, in the same manner as Fig. 2, the protection afforded to a bacterial filter by various commercially available deep bed fiber filters in an apparatus similar to that of Fig. 1.

## Claims

1. An apparatus for administering respirable pharmaceutical particles to at least one lung of a patient, comprising: a ventilator for cyclically forcing oxygen-rich air into at least one lung of said patient and collecting oxygen-depleted air exhaled by said patient;
an inspiratory limb connected to said ventilator for carrying said oxygen-rich air from said ventilator to said patient;
an expiratory limb connected to said ventilator for carrying said oxygen-depleted air exhaled by said patient to said ventilator;
a nebulizer connected to said inspiratory limb for introducing respirable pharmaceutical particles into said oxygen-rich air; and
a prefilter connected to said expiratory limb between said patient and said ventilator for removing respirable pharmaceutical particles from said oxygen-depleted air exhaled by said patient.

2. An apparatus according to claim 1, further comprising a bacterial filter connected to said expiratory limb between said prefilter and said ventilator.

3. An apparatus according to claim 1, wherein said prefilter comprised a deep-bed filter.

4. An apparatus according to either of claim 1 wherein said prefilter comprises a deep-bed filter selected from the group consisting of fiber filters and porous foam filters.

5. An apparatus according to claim 1, wherein said nebulizer introduces from 10 to 50 µL of respirable pharmaceutical particles as an aerosol into each liter of oxygen-rich air.

6. An apparatus according to claim 1, wherein said prefilter comprises a resistance of not more than 4 centimeters of water at an air flow of 100 liters per minute.

7. An apparatus according to claim 1, wherein said prefilter has a resistance of not more than 2 centimeters of water at an air flow of 100 liters per minute.

8. An apparatus according to claim 1, wherein said expiratory limb has a total resistance of not more than 4 centimeters of water at an air flow of 100 liters per minute.

9. An apparatus according to claim 1, wherein said respirable pharmaceutical particles are comprised of a surfactant formulation.

10. A method of administering respirable pharmaceutical particles to at least one lung of a patient, comprising:
providing a ventilator for cyclically forcing oxygen-rich air into at least one lung of said patient and collecting oxygen-depleted air exhaled by said patient;
introducing respirable pharmaceutical particles into said oxygen-rich air; and
removing respirable pharmaceutical particles from said oxygen-depleted air exhaled by said patient before said oxygen-depleted air is collected by said ventilating terms.
